# EUROPEAN PATENT APPLICATION

(11) **EP 1 655 042 A1**
(43) Date of publication of application: **10.05.2006**
(21) Application number: 04396075.6
(22) Date of filing: 02.11.2004
(51) Int. Cl.: A61L 15/18, A61L 27/10, A61L 31/02, A61K 9/14

(54) **A medical device**

(71) Applicant: Vivoxid Oy, 20520 Turku (FI)
(72) Inventor: Järveläinen, Hannu, 20900 Turku (FI); Laato, Matti, 21270 Nousiainen (FI); Salonen, Jukka, 20100 Turku (FI); Vedel, Erik, 21600 Parainen (FI)
(74) Representative: Suominen, Kaisa Liisa

(57) **Abstract**

The invention relates to a device comprising a bioactive glass composition comprising SiO₂, Na₂O, CaO, K₂O, MgO, B₂O₃ and P₂O₅, wherein the amount of SiO₂ is 50-65 wt-% of the final total weight, Na₂O is 5-26 wt-% of the final total weight, CaO is 10-25 wt-% of the final total weight, K₂O is 0-15 wt-% of the final total weight, MgO is 0-6 wt-% of the final total weight, B₂O₃ is 0-4 wt-% of the final total weight, and P₂O₅ is 0-4 wt-% of the final total weight, provided that the total amount of Na₂O and K₂O is 10-30 wt-% of the final total weight. The invention also relates to the use of said composition for treating lesions associated with compromised or poor vascularisation and for preventing avascular fibrosis.

## Description

### FIELD OF THE INVENTION

This invention relates to a medical device. The invention also relates to the use for treating lesions associated with compromised or poor vascularisation.

### BACKGROUND OF THE INVENTION

The publications and other materials used herein to illuminate the background of the invention, and in particular, the cases to provide additional details respecting the practice, are incorporated by reference.

The treatment of lesions, for example skin lesions, associated with compromised or poor vascularisation or blood perfusion is a demanding and difficult area of medicine. Improvement of angiogenesis, i.e. the new capillary blood vessel formation, is particularly important when natural healing is slow or is rendered difficult by a number of negative factors associated with e.g. infection of the wound, impeded blood flow, bums or other types of tissue damage/injury, medical treatment with cell poisons or steroids of various kind, or in cases when the patient suffers from chronic disorders with concomitant impairment of normal wound healing, i.e. when he or she is bedridden for prolonged periods of time, is of old age, has a cancer disease giving rise to serious nutritional deficiencies, has chronic inflammatory conditions of the intestine, has diabetes, is affected by conditions caused by atherosclerosis or venous diseases, and comparable types of conditions.

Currently the treatment of skin lesions mentioned above can be very problematic and places an enormous drain on the health resources. Recently various new approaches have been tested, including topical growth factors (e.g. platelet-derived growth factor, transforming growth factor β, granulocyte macrophage-colony stimulating factor), autologous skin grafts and bioengineered skin equivalents (e.g. Alloderm®, Integra®, Dermagraft-TC®, Apligraft®). Furthermore, interest is presently focused on angiogenesis induced e.g. by vascular endothelial growth factor delivered by gene therapy.

Another problem encountered in this field is the formation of an avascular fibrous capsule around an implanted biomaterial device and the masstransfer resistance associated with this fibrous capsule.

The use of bioactive glasses in medicine is now widely known. In this application, by bioactive glass it is meant a material that has been designed to induce specific biological activity in body tissues.

Bioactive glasses react in aqueous systems and develop layers on their surfaces resulting in bonding between the device and the host tissue, but also release some of its component ions into the tissue. These ions can drift several hundred micrometers from the device surface and stimulate cell growth and angiogenesis as described in this application. Unlike most other bioactive materials, the rate of chemical reactions of bioactive glasses can be easily controlled by changing the chemical composition of the glass.

### OBJECTS AND SUMMARY OF THE INVENTION

The object of this invention is therefore to provide a medical device that can be used for treatment of lesions associated with compromised or poor vascularisation. Examples of such lesions are given above.

A typical medical device according to the present invention comprises a bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
   provided that the total amount of Na₂O and K₂O is 10-30 wt-% of the final total weight.

The present invention also relates to the medical use of said bioactive glass composition for treating lesions associated with compromised or poor vascularisation.

### DETAILED DESCRIPTION OF THE INVENTION

The present invention thus relates to a medical device comprising a bioactive glass composition as disclosed above. The medical device is thus a vascularisation and healing promoting device. Other embodiments of the present invention are disclosed in the dependent claims.

The present invention provides a medical device that can be used for treatment of lesions associated with compromised or poor vascularisation as well as for induction of neovascularised fibrotic capsule. The device can thus be used for treatment of lesions caused by conditions due to lack of vascularisation or due to pathological changes of the vascularisation. It can also be used in association with drug delivery devices, cell encapsulation devices, tissue engineered neo-organ formation and implanted senseors that need proper vascularisation in order to function. The important point is not only the quantity of blood vessels (capillaries, arterioles and venules), but also their structural quality at the compromised site.

The bioactive glasses used in the present invention dissolve congruently in liquids, such as body fluids. Such bioactive glasses provide a burst of ions when in contact with the lesion, i.e., in contact with the tissue to be engineered.

By compromised or poor vascularisation at the site of a lesion it is meant blood perfusion that has either deteriorated from the original, normal vascularisation, or that has not developed correctly, that is, up to the normal level, i.e., lacking vascularisation. By normal vascularisation it is meant the physiological vascularisation normally taking place in a healthy person.

The device according to the present invention thus comprises said bioactive glass composition. This means that the device may also comprise other materials. It may for example comprise additives. Such additives are for example biologically active agents, cellulose materials, cotton, other bioactive glasses, polymers or other structure supporting materials. Said cellulose materials may for example be carboxymethyl cellulose, biomineralized cellulose or biomineralizable cellulose. Said polymer may be any polymer suitable *per se,* such as polyolefins or polyesters.

The bioactive glass composition may be used in any suitable form, such as in the form of fibers or particles. The composition may be used in the form of a woven or nonwoven tissue. When fibers are used, their diameter is typically in the range of 10 - 100 µm. On the other hand, when particles are used, the diameter of the particles is typically below 90 µm. When particles are used, they may be bound together by a suitable binding agent, such as collagen, alginate, polysaccharides such as starch or hexose sugars, or polyvinylpyrrolidone. It is also naturally possible to use several different bioactive glass compositions in one device.

The device according to the present invention may have any size and shape desired. It may be in the form of granules, powder, paste, sheet (woven or nonwoven), bandage, mat, pad or band-aid. It may be for example a large sheet used for treating pressure sores or a small sheet or in powder form for treating wounds associated with diabetes and/or other conditions exhibiting compromised or poor vascularisation. When the bioactive glass is used in powder form, it may be then covered with any known bandage.

In the compositions above, the amount of different oxides is given as weight percent of the final total weight.

It is obvious to a person skilled in the art that the amounts of the oxides can be freely chosen within the above-mentioned limits. Indeed, the amount of SiO₂ can be for example 51.5, 52, 53.5, 55, 56, 58, 61, 62.5 or 65 wt-% of the final total weight, the amount of Na₂O can be for example 5, 5.5, 6.2, 7, 7.3, 7.7, 8, 8.5, 9, 12, 15, 16.5, 18, 20.4, 24, 25.1 or 26 wt-% of the final total weight, the amount of CaO can be for example 10, 12.5, 14.7, 15, 16.5, 17, 20.3, 21, 21.4, 21.7, 22, 22.6, 23 or 25 wt-% of the final total weight, the amount of K₂O can be for example 0, 1.2, 2.5, 5, 5.5, 6.2, 7, 7.3, 8, 10, 10.5, 10.6, 11, 11.3, 11.7, 12, 14.2 or 15 wt-% of the final total weight, the amount of MgO can be for example 0, 0.5, 1, 1.3, 1.9, 2.4, 2.7, 3.5, 4, 4.5, 5.2 or 6 wt-% of the final total weight, the amount of B₂O₃ can be for example 0, 0.4, 0.6, 0.9, 1, 1.2, 2.7, 3.5 or 4 wt-% of the final total weight, and the amount of P₂O₅ can be for example 0, 0.5, 0.7, 1, 1.2, 2.5, 3.2 or 4 wt-% of the final total weight.

The bioactive glass composition used in the present invention can be made for example either by melting or by sol-gel process. The latter leads to a more porous glass than the melt-process.

According to an embodiment of the present invention, the bioactive glass has the following composition:
SiO₂ is 52-54 wt-% of the final total weight,
Na₂O is 5-7 wt-% of the final total weight,
CaO is 21-23 wt-% of the final total weight,
K₂O is 10-12 wt-% of the final total weight,
MgO is 4-6 wt-% of the final total weight,
B₂O₃ is 0-2 wt-% of the final total weight, and
P₂O₅ is 0-1 wt-% of the final total weight.

According to another embodiment of the present invention, the bioactive glass has the following composition:
SiO₂ is 59-61 wt-% of the final total weight,
Na₂O is 24-26 wt-% of the final total weight,
CaO is 10-12 wt-% of the final total weight,
K₂O is 0-1 wt-% of the final total weight,
MgO is 0-1 wt-% of the final total weight,
B₂O₃ is 0-3 wt-% of the final total weight, and
P₂O₅ is 1-4 wt-% of the final total weight.

According to yet another embodiment of the present invention, the bioactive glass has the following composition:
SiO₂ is 52-54 wt-% of the final total weight,
Na₂O is 22-24 wt-% of the final total weight,
CaO is 19-21 wt-% of the final total weight,
K₂O is 0-1 wt-% of the final total weight,
MgO is 0-1 wt-% of the final total weight,
B₂O₃ is 0-1 wt-% of the final total weight, and
P₂O₅ is 0-1 wt-% of the final total weight.

According to an especially preferable embodiment, the bioactive glass has the composition of
SiO₂ is 53 wt-% of the final total weight,
Na₂O is 23 wt-% of the final total weight,
CaO is 20 wt-% of the final total weight and
P₂O₅ is 4 wt-% of the final total weight.

According to a still further embodiment of the present invention, the bioactive glass has the following composition:
SiO₂ is 52-61 wt-% of the final total weight,
Na₂O is 5-24 wt-% of the final total weight,
CaO is 10-23 wt-% of the final total weight,
K₂O is 0-12 wt-% of the final total weight,
MgO is 0-6 wt-% of the final total weight,
B₂O₃ is 0-3 wt-% of the final total weight, and
P₂O₅ is 0-4 wt-% of the final total weight,
provided that the total amount of Na₂O and K₂O is 10-30 wt-% of the final total weight.

The present invention also relates to a bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
provided that the total amount of Na₂O and K₂O is 10-30 wt-% of the final total weight, for treating lesions associated with compromised vascularisation.

The present bioactive glass composition can thus be used for treating lesions associated with compromised or poor vascularisation or promoting vascularisation of tissues associated with implanted biomaterial. The lesions include skin lesions or any other lesion or damaged tissue that exhibit delayed healing due to the lack of proper vascularisation or tendency for developing avascular fibrosis. In a typical treatment the lesion is firstly decontaminated with any known products, and then a device according to the present invention is applied on the lesion and preferably attached with a gauze or the like. The device is left on place for the time needed for healing or changed at constant intervals, such as every day or once a week, depending on the characteristics of the lesion that is treated.

The invention further relates to a use of a bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
provided that the total amount of Na₂O and K₂O is 10-30 wt-% of the final total weight, for the manufacture of a medical device for treating lesions associated with compromised or poor vascularisation.

The details given above in connection with the device apply also with respect to the uses.

In this specification, except where the context requires otherwise, the words "comprise", "comprises" and "comprising" means "include", "includes" and "including", respectively. That is, when the invention is described or defined as comprising specified features, various embodiments of the same invention may also include additional features.

The invention is described below in greater detail by the following, nonlimiting drawings.

### BRIEF DESCRIPTION OF THE DRAWINGS

Figure 1 schematically illustrates a medical device according to a first embodiment of the present invention.
Figure 2 illustrates the results of Example 5.

### DETAILED DESCRIPTION OF THE DRAWINGS

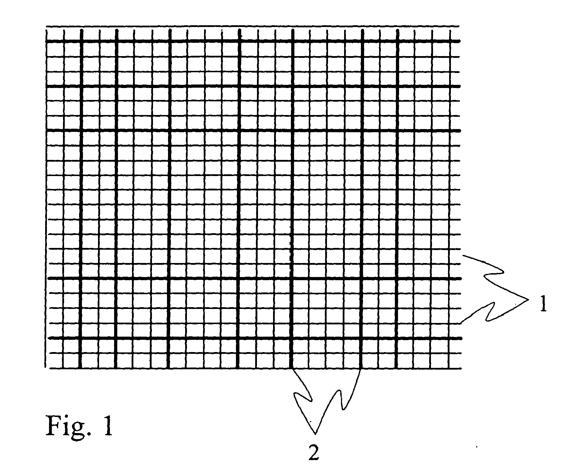

Figure 1 schematically illustrates a medical device according to a first embodiment of the present invention. In this embodiment, the medical device is constituted of a woven tissue consisting essentially of fibers in two directions. The fibers 1 are made of a different bioactive glass composition that the fibers 2.

### EXPERIMENTAL PART

### Example 1

A composition consisting of:
158.25 g of SiO₂,
14.55 g of CaH(PO₄)x2H₂O,
98.63 g of CaCO₃,
104.60 g of Na₂CO₃,
16.51 g of K₂CO₃, and
4.50 g of MgO.
was heated to a temperature of 1360 °C and maintained at this temperature for a period of three hours. The melted composition wherein the carbonates had reacted forming oxides was allowed to cool down to ambient temperature overnight and the solid glass was crushed.

The crushed glass material was reheated to a temperature of 1360 °C and maintained in this temperature for a period of three hours. The resulting glass was cast into a mold and allowed to cool down to ambient temperature. 300 g of bioactive glass according to the present invention was obtained. The composition of the glass was the following:
SiO₂ 52.75 wt-%,
P₂O₅ 2.00 wt-%,
CaO 220.00 wt-%,
Na₂O 20.00 wt-%,
K₂O 3.75 wt-%, and
MgO 1.50 wt-%.

### Example 2

A composition consisting of:
159.00 g of SiO₂,
29.10 g of CaH(PO₄)x2H₂O,
90.16 g of CaC03 and
118.0 g of Na₂CO₃
was heated to a temperature of 1360 °C and maintained at this temperature for a period of three hours. The melted composition wherein the carbonates had reacted forming oxides was allowed to cool down to ambient temperature overnight and the solid glass was crushed.

The crushed glass material was reheated to a temperature of 1360 °C and maintained in this temperature for a period of three hours. The resulting glass was cast into a mold and allowed to cool down to ambient temperature. 300 g of bioactive glass according to the present invention was obtained. The composition of the glass was the following:
SiO₂ 53 wt-%,
P₂O₅ 4 wt-%,
CaO 20 wt-% and
Na₂O 23 wt-%,

### Example 3

A composition consisting of:
168.00 g of SiO₂,
14.55 g of CaH(PO₄)x2H₂O,
74.85 g of CaCO₃,
128.26 g of Na₂CO₃, and
10.66 g of H₃BO₃
was heated to a temperature of 1360 °C and maintained at this temperature for a period of three hours. The melted composition wherein the carbonates had reacted forming oxides was allowed to cool down to ambient temperature overnight and the solid glass was crushed.

The crushed glass material was reheated to a temperature of 1360 °C and maintained in this temperature for a period of three hours. The resulting glass was cast into a mold and allowed to cool down to ambient temperature. 300 g of bioactive glass according to the present invention was obtained. The composition of the glass was the following:
SiO₂ 56 wt-%,
P₂O₅ 2 wt-%,
CaO 15 wt-%,
Na₂O 25 wt-%, and
B₂O₃ 2 wt-%.

### Example 4

179.10 g of SiO₂,
18.19 g of CaH(PO₄)x2H₂O,
48.32 g of CaCO₃,
130.82 g of Na₂CO₃, and
6.93 g of H₃BO₃
was heated to a temperature of 1360 °C and maintained at this temperature for a period of three hours. The melted composition wherein the carbonates had reacted forming oxides was allowed to cool down to ambient temperature overnight and the solid glass was crushed.

The crushed glass material was reheated to a temperature of 1360 °C and maintained in this temperature for a period of three hours. The resulting glass was cast into a mold and allowed to cool down to ambient temperature. 300 g of bioactive glass according to the present invention was obtained. The composition of the glass was the following:
SiO₂ 59,7 wt-%,
P₂O₅ 2,5 wt-%,
CaO 11 wt-%,
Na₂O 25,5 wt-% and
B₂O₃ 1,3 wt-%.

Any of the above-mentioned bioactive glasses can be used for drawing of a fiber by standard methods.

### Example 5

The effect of the bioactive glass composition A (disclosed in Example 2) on stimulating angiogenesis was studied by using a standardized experimental wound model, i.e. in subcutaneously implanted viscose cellulose sponges in mice.

Viscose cellulose sponges (manufactured by Cellomeda Oy, Turku, Finland) was used as a framework matrix to study tissue repair. The material was cut into rectangular pieces, 20 mm long and 5 mm in diameter. The sponges were decontaminated by boiling for 30 minutes in physiological saline and the implantations were performed with strictly aseptic techniques. C57BL mice were anesthetized and a 2 cm long incision was made in the dorsal midline at the caudal portion of the back. Each mouse received two sponges that were implanted longitudinally under the skin, cephaled from the incision. During the experiments, the animals received a normal mouse diet and water *ad libitum* and were housed individually in cages in the animal quarters.

Viscose cellulose sponges were filled with 100 µl of a suspension containing 700 µl 0.9 % NaCl solution, 0.5 g of the glass of Example 2 and 1 g of hexose sugars, or with 0.9 % NaCl solution alone. Seventeen and 24 days after sponge implantations, the mice were sacrificed, and the sponges were harvested and analyzed for vascularity by counting the number of all erythrocyte-containing blood vessels (capillaries) in formalin-fixed, paraffin-embedded and haematoxylin and eosin stained histological sections of sponge specimens using light microscope (magnification 40x) as described in the publication Puolakkainen et al., 2003 (Puolakkainen P, Bradshaw AD, Kyriakides TR, Reed M, Brekken R, Wight T, Bornstein P, Ratner B, Sage EH. Compromised production of extracellular matrix in mice lacking secreted protein, acidic and rich in cysteine (SPARC) leads to a reduced foreign body reaction to implanted biomaterials. Am. J. Pathol. 2003; 162: 627-635).

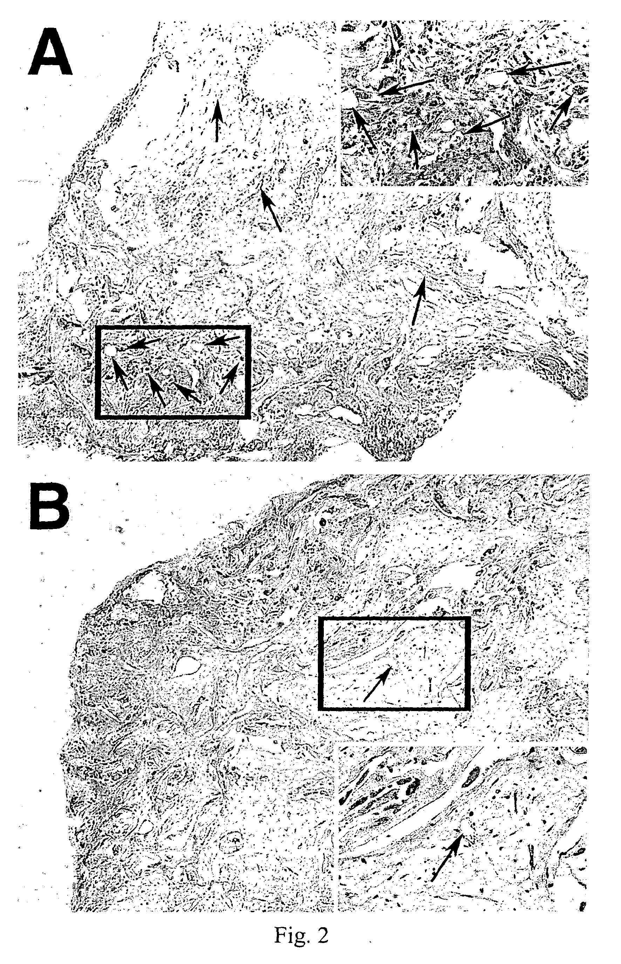

The results demonstrated that the sponges filled with bioactive glass and hexose sugars containing suspension exhibited markedly more blood vessels compared to the sponges filled with the suspension alone (see Figure 2, in which representative pictures on sponges filled with bioactive glass containing suspension (panel A) or with the suspension alone (panel B) at the 24 day time point are shown. The arrows in the pictures indicate erythrocyte containing blood vessels). The above results indicate that bioactive glass has a stimulatory effect on new capillary blood vessel formation (i.e. angiogenesis), and thereby, bioactive glass is believed to be of great value when new strategies e.g. for impaired wound healing are designed.

## Claims

1. A medical device consisting essentially of a bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
provided that the total amount of Na₂O and K₂O is 10-30 wt-% of the final total weight.

2. Medical device according to claim 1, **characterized in that** the amount of
SiO₂ is 52-54 wt-% of the final total weight,
Na₂O is 5-7 wt-% of the final total weight,
CaO is 21-23 wt-% of the final total weight,
K₂O is 10-12 wt-% of the final total weight,
MgO is 4-6 wt-% of the final total weight,
B₂O₃ is 0-2 wt-% of the final total weight, and
P₂O₅ is 0-1 wt-% of the final total weight.

3. Medical device according to claim 1, **characterized in that** the amount of
SiO₂ is 59-61 wt-% of the final total weight,
Na₂O is 24-26 wt-% of the final total weight,
CaO is 10-12 wt-% of the final total weight,
K₂O is 0-1 wt-% of the final total weight,
MgO is 0-1 wt-% of the final total weight,
B₂O₃ is 0-3 wt-% of the final total weight, and
P₂O₅ is 1-4 wt-% of the final total weight.

4. Medical device according to claim 1, **characterized in that** the amount of
SiO₂ is 52-54 wt-% of the final total weight,
Na₂O is 22-24 wt-% of the final total weight,
CaO is 19-21 wt-% of the final total weight,
K₂O is 0-1 wt-% of the final total weight,
MgO is 0-1 wt-% of the final total weight,
B₂O₃ is 0-1 wt-% of the final total weight, and
P₂O₅ is 0-1 wt-% of the final total weight.

5. Medical device according to claim 1, **characterized in that** it further consists of additives selected from the group consisting of biologically active agents, cellulose materials, cotton, other bioactive glasses and polymers.

6. A bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
provided that the total amount of Na₂O and K₂O is 10-30 wt-% of the final total weight, for treating lesions associated with compromised or poor vascularisation.

7. A bioactive glass composition according to claim 6, **characterized in that** the lesion is a skin lesion.

8. Use of a bioactive glass composition comprising SiO₂, Na₂O, CaO, K₂O, MgO, B₂O₃ and P₂O₅, wherein the amount of
SiO₂ is 50-65 wt-% of the final total weight,
Na₂O is 5-26 wt-% of the final total weight,
CaO is 10-25 wt-% of the final total weight,
K₂O is 0-15 wt-% of the final total weight,
MgO is 0-6 wt-% of the final total weight,
B₂O₃ is 0-4 wt-% of the final total weight, and
P₂O₅ is 0-4 wt-% of the final total weight,
provided that the total amount of Na₂O and K₂O is 10-30 wt-% of the final total weight, for the manufacture of a medical device for treating lesions associated with compromised or poor vascularisation.
